(19) Europäisches Patentamt
European Patent Office
Office européen des brevets

(11) Numéro de publication : 0 022 735
B1

(12) FASCICULE DE BREVET EUROPÉEN

(45) Date de publication du fascicule du brevet : 28.04.82

(51) Int. Cl.³ : C 07 C 45/49, C 07 C 47/06

(21) Numéro de dépôt : 80420084.8

(22) Date de dépôt : 02.07.80

(54) **Procédé de préparation de l'acétaldéhyde.**

(30) Priorité : 04.07.79 FR 7917927

(43) Date de publication de la demande : 21.01.81 (Bulletin 81/03)

(45) Mention de la délivrance du brevet : 28.04.82 Bulletin 82/17

(84) Etats contractants désignés : AT BE CH DE FR GB IT LI LU NL SE

(56) Documents cités :
EP - A - 0 011 042
US - A - 3 356 734

(73) Titulaire : RHONE-POULENC INDUSTRIES
22 Avenue Montaigne
F-75008 Paris (FR)

(72) Inventeur : Gauthier-Lafaye, Jean
21, rue Duguesclin
F-69006 Lyon (FR)
Inventeur : Perron, Robert
La Pecolière
F-69390 Charly (FR)

(74) Mandataire : Varniere-Grange, Monique et al
RHONE-POULENC RECHERCHES Centre de Recherches de Saint-Fons Service Brevets B.P. 62
F-69190 Saint-Fons (FR)

EP 0 022 735 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Procédé de préparation de l'acétaldéhyde

La présente invention a pour objet un procédé de préparation de l'acétaldéhyde par carbonylation du méthanol en présence d'hydrogène.

L'acétaldéhyde est un intermédiaire de grand intérêt dans l'industrie chimique. Il est utile, notamment pour la fabrication de l'acide acétique et de l'anhydride acétique. (Cf. « Encyclopedia of Chemical Technology » KIRK - OTHMER. 3e Edition, volume 1, page 97 et suivantes).

Des procédés industriels ont été développés pour produire de l'acétaldéhyde. Le procédé le plus utilisé actuellement est l'oxydation directe de l'éthylène. Toutefois, cet hydrocarbure étant issu du pétrole, il peut devenir plus économique de choisir des matières premières issues du gaz de synthèse, telles que le méthanol.

L'utilisation du méthanol comme matière première dans la synthèse de produits qui, traditionnellement, proviennent à l'échelle industrielle de l'éthylène, a constitué l'objet et continue d'être l'objet de nombreuses recherches. Ces recherches visent essentiellement l'application des techniques de carbonylation, c'est-à-dire de la réaction du monoxyde de carbone sur le méthanol, éventuellement en présence d'hydrogène.

C'est ainsi que de nombreux travaux antérieurs ont porté sur l'homologation du méthanol, c'est-à-dire sur la production d'éthanol par carbonylation du méthanol.

Il est bien connu que le méthanol réagit avec un mélange d'oxyde de carbone et d'hydrogène (1 : 1) à 185 °C et sous 360 atmosphères en présence de dicobaltoctacarbonyle. Dans ces conditions, on obtient un mélange de divers produits, renfermant de l'éthanol avec une sélectivité relativement faible. (Cf. WENDER et coll. « Science » volume 113 page 206, 1951).

D'autres auteurs ont montré que lorsque la réaction d'homologation est conduite à 200 °C sous 200 à 350 atmosphères en présence de diacétate de cobalt, le rendement en éthanol peut être amélioré en opérant simultanément en présence d'un promoteur iodé ($I_2$ ou $CH_3I$) et d'un mélange gazeux riche en oxyde de carbone. (Cf. BERTY et coll. « Chem. Tech. » volume 5, pages 260-266, 1956).

D'autres progrès vers la production d'éthanol ont pu être réalisés par adjonction au système catalytique précédent d'un composé phosphoré soluble dans le méthanol (Cf. Brevet français n° 1 341 840), par introduction d'halogénures de ruthénium ou d'osmium (Cf. brevet des Etats-Unis d'Amérique n° 3 285 948), ou bien par addition d'une phosphine tertiaire et d'un hydrocarbure comme solvant (Cf. brevet français n° 2 314 166).

Néanmoins, ces procédés ne sont pas applicables à l'échelle industrielle : ils ne permettent pas d'atteindre des sélectivités élevées en éthanol et par suite nécessitent la mise en place d'installations complexes pour séparer les divers constituants du mélange obtenu ce qui alourdit de manière inacceptable l'économie globale d'un tel procédé.

Partant de cette constatation, d'autres auteurs se sont orientés vers la production d'acétaldéhyde. C'est ainsi qu'il a été mentionné que la présence d'une quantité de cobalt inférieure à 2 millimoles par mole de méthanol dans le système catalytique (cobalt-halogène) favorise la conversion du méthanol en acétaldéhyde (Cf. brevet des Etats-Unis d'Amérique n° 3 356 734).

Effectivement, lorsque l'on réalise la carbonylation du méthanol selon la technique objet du brevet américain précité, à 185 °C sous 300 à 400 atm avec un rapport molaire $CO/H_2$ de 1.4 pendant 2 heures, on obtient environ 130 g d'acétaldéhyde par litre de milieu réactionnel et par heure, pour une productivité de l'ordre de 70 g d'acétaldéhyde par heure et par gramme de cobalt engagé dans la réaction, compte tenu du fait que le diméthoxyéthane formé est une source potentielle de l'acétaldéhyde.

Des travaux plus récents (Cf. demandes de brevet japonais 77/136 111 et 77/133 914) ont montré que les résultats atteints à l'aide de ce système catalytique peuvent être sensiblement améliorés par adjonction au système (Cobalt, Iode) d'une quantité importante d'un composé phosphoré ou d'un composé de l'arsenic, de l'antimoine ou du bismuth. Néanmoins, la productivité en acétaldéhyde par rapport au cobalt engagé reste faible, malgré les pressions élevées mises en œuvre. En outre selon les procédés connus, une partie non négligeable du méthanol est convertie en butanol, butanal et buténal, produits dont la valorisation est aléatoire, et qui grèvent l'économie globale du procédé en nécessitant des étapes supplémentaires de séparation. D'autre part, la possibilité d'exploitation industrielle de tels procédés est compromise par la pression élevée nécessaire pour atteindre des productivités horaires acceptables. Des auteurs (Cf. T. MIZOROKI et Coll., Bull. Chem. Soc. Jap. Vol. 52 (2), 479 (1979)) ont étudié la carbonylation du méthanol en présence de cobalt et d'iodure de méthyle, dans le benzoate de méthyle et sous 150 bars environ. Ils ont confirmé que dans ces conditions l'adjonction de faibles quantités de ruthénium accroît sensiblement la sélectivité en éthanol, au détriment de l'acétaldéhyde. Ils ont également montré le rôle prépondérant joué par l'iodure de sodium, dans cette réaction. Néanmoins, la productivité horaire en acétaldéhyde ne dépasse pas 65 g/h × l de milieu réactionnel et n'atteint que 14 g/h × g (Co) engagé à la réaction.

Il a maintenant été trouvé de manière inattendue que l'addition de faibles quantités de ruthénium à un système catalytique renfermant une faible teneur en cobalt permet d'augmenter considérablement les performances dudit système, ce qui permet la production sélective d'acétaldéhyde par hydrocarbonylation du méthanol, avec une productivité horaire remarquable, sous une pression totale aussi faible que 100 bars.

La présente invention a donc pour objet un procédé d'hydrocarbonylation du méthanol en présence simultanée d'au moins un halogénure ionique, d'au moins un halogénure d'alkyle, d'au plus 50 milliatomes-grammes de cobalt par litre de milieu réactionnel et d'au plus 2 atomes-grammes de ruthénium par atome-gramme de cobalt présent dans le milieu, la température étant au moins égale à 180 °C.

Le procédé selon l'invention exige la mise en œuvre d'au moins un halogénure ionique. Par halogénure ionique on entend les chlorures, bromures, ou, de préférence, les iodures minéraux ou organiques dont les cations sont choisis parmi les cations des métaux alcalins, les cations des métaux alcalino-terreux et les cations ammonium ou phosphonium quaternaire représentés par les formules I à III ci-après :

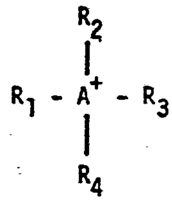
(I)

dans laquelle A représente un atome d'azote ou de phosphore, $R_1$, $R_2$, $R_3$ et $R_4$, pouvant être identiques ou différents représentent l'hydrogène ou, de préférence, des radicaux organiques dont la valence libre est portée par un atome de carbone, deux quelconques de ces divers radicaux pouvant éventuellement former ensemble un radical unique divalent.

Plus spécifiquement $R_1$, $R_2$, $R_3$ et $R_4$ peuvent représenter des radicaux alkyles linéaires ou ramifiés, des radicaux cycloalkyles, aralkyles (par exemple benzyle) ou aryles monocycliques, ayant au plus 16 atomes de carbone, pouvant le cas échéant être substitués par 1 à 3 radicaux alkyles ayant de 1 à 4 atomes de carbone ; deux des radicaux $R_1$ à $R_4$ pouvant éventuellement former ensemble un radical unique divalent — alkylène ou alcénylène — comportant 3 à 6 atomes de carbone (par exemple un radical tétraméthylène ou hexaméthylène) et le cas échéant 1 ou 2 doubles liaisons éthyléniques, ledit radical pouvant porter 1 à 3 substituants alkyles ayant de 1 à 4 atomes de carbone

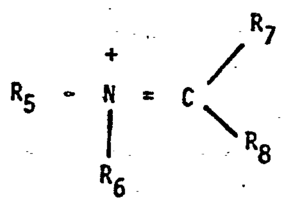
(II)

dans laquelle $R_5$, $R_6$, $R_7$ et $R_8$ identiques ou différents représentent des radicaux alkyles ayant de 1 à 4 atomes de carbone, l'un des radicaux $R_7$ ou $R_8$ pouvant en outre représenter l'hydrogène, $R_7$ et $R_8$ pouvant éventuellement former ensemble un radical unique divalent alkylène comportant de 3 à 6 atomes de carbone, tétraméthylène ou hexaméthylène par exemple ; $R_6$ et $R_7$ ou $R_8$ peuvent former ensemble un radical unique divalent — alkylène ou alcénylène — comportant 4 atomes de carbone et le cas échéant 1 ou 2 doubles liaisons éthyléniques, l'atome d'azote étant alors inclus dans un hétérocycle, pour constituer, par exemple, un cation pyridinium

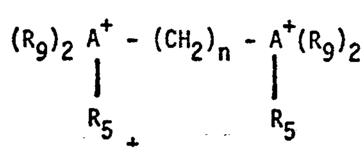
(III)

dans laquelle $R_5$ et $A^+$ ont la signification donnée précédemment, $R_9$ pouvant être identique à $R_5$ représente un radical alkyle ayant de 1 à 4 atomes de carbone ou un radical phényle, et n est un entier compris entre 1 et 10 ($1 \leq n \leq 10$) et de préférence entre 1 et 6 ($1 \leq n \leq 6$). A titre d'exemple d'halogénures d'ammonium quaternaire convenant à la mise en œuvre du présent procédé on peut citer les chlorures, les bromures et, plus particulièrement les iodures

de tétraméthylammonium,
de triéthylméthylammonium,
de tributylméthylammonium,
de triméthyl (n-propyl) ammonium,
de tétraéthylammonium,
de tétrabutylammonium,

de dodécyltriméthylammonium,
de benzyltriméthylammonium,
de benzyldiméthylpropylammonium,
de benzyldiméthyloctylammonium,
de diméthyldiphénylammonium,
de méthyltriphénylammonium,
de N,N-diméthyl-triméthylénammonium,
de N,N-diéthyl-triméthylénammonium,
de N,N-diméthyl-tétraméthylénammonium,
de N,N-diéthyl-tétraméthylénammonium,
de N-méthylpyridinium,
de N-éthylpyridinium,
de N-méthylpicolinium,

A titre d'exemple d'halogénures de phosphonium quaternaire convenant également à la mise en œuvre du présent procédé, on peut citer les chlorures, les bromures et, plus particulièrement les iodures

de tétraméthylphosphonium,
d'éthyltriméthylphosphonium,
de triméthylpentylphosphonium,
d'octyltriméthylphosphonium,
de dodécyltriméthylphosphonium,
de triméthylphénylphosphonium,
de diéthyldiméthylphosphonium,
de dicyclohexyldiméthylphosphonium,
de diméthyldiphénylphosphonium,
de cyclohexyltriméthylphosphonium,
de triéthylméthylphosphonium,
de méthyl-tri (isopropyl) phosphonium,
de méthyl-tri (n-propyl) phosphonium,
de méthyl-tri (n-butyl) phosphonium,
de méthyl-tris (méthyl-2 propyl) phosphonium,
de méthyltricyclohexylphosphonium,
de méthyltriphénylphosphonium,
de méthyltribenzylphosphonium,
de méthyl-tris (méthyl-4 phényl) phosphonium,
de méthyltrixylylphosphonium,
de diétylméthylphénylphosphonium,
de dibenzylméthylphénylphosphonium,
d'éthyltriphénylphosphonium,
de tétraéthylphosphonium,
d'éthyl-tri (n-propyl) phosphonium,
de triétylpentylphosphonium,
d'éthyltriphénylphosphonium,
de n-butyl-tri-(n-propyl) phosphonium,
de butyltriphénylphosphonium,
de benzyltriphénylphosphonium,
de (β-phénylléthyl) diméthylphénylphosphonium,
de tétraphénylphosphonium,
de triphényl (méthyl-4 phényl) phosphonium.

La nature précise du cation ammonium ou phosphonium quaternaire n'est pas fondamentale dans le cadre du présent procédé. Le choix parmi ces composés est davantage orienté par des considérations d'ordre pratique, telles que la solubilité dans le milieu réactionnel, la disponibilité et la commodité d'emploi.

A ce titre, les halogénures d'ammonium ou de phosphonium quaternaire représentés soit par la formule (I) dans laquelle l'un quelconque des radicaux $R_1$ à $R_4$ est choisi parmi les radicaux alkyles linéaires ayant de 1 à 4 atomes de carbone, soit par les formules (II) ou (III) dans laquelle $R_5$ (ou $R_6$) est également un radical alkyle ayant de 1 à 4 atomes de carbone, conviennent particulièrement bien.

En outre, parmi les halogénures d'ammonium on préfère ceux dont les cations correspondent à la formule I dans laquelle tous les radicaux $R_1$ à $R_4$ sont choisis parmi les radicaux alkyles linéaires ayant de 1 à 4 atomes de carbone et dont au moins trois d'entre eux sont identiques.

De même, parmi les halogénures de phosphonium quaternaire, on préfère ceux dont les cations correspondent à la formule (I) dans laquelle l'un quelconque des radicaux $R_1$ à $R_4$ représente un radical alkyle linéaire ayant de 1 à 4 atomes de carbone, les trois autres radicaux étant identiques et choisis parmi

les radicaux phényle, tolyle ou xylyle.

Les iodures de phosphonium quaternaire et plus particulièrement ceux dont les cations correspondent à la formule (I) ci-avant, dans laquelle l'un des radicaux $R_1$ à $R_4$ est un radical alkyle ayant de 1 à 4 atomes de carbone, les trois autres radicaux étant identiques et choisis parmi les radicaux phényl, tolyle ou xylyle, constituent une classe d'halogénures ioniques particulièrement commodes pour la mise en œuvre de la présente invention.

Un mode préféré de réalisation de la présente invention comprend l'utilisation d'iodures des métaux alcalins ou alcalino-terreux, tels que : LiI, NaI, KI, CsI, $CaI_2$ et $MgI_2$. De préférence on utilise un ou plusieurs iodures des métaux alcalins et de manière encore plus avantageuse, NaI ou KI.

Selon la présente invention, le rapport molaire $X^-/Co$, $X^-$ provenant de l'halogénure ionique doit être au moins égal à 5. Il n'est pas souhaitable que ce rapport dépasse la valeur de 400. Des résultats très satisfaisants sont obtenus pour un rapport $X^-/Co$ de l'ordre de 5 à 150.

Le procédé selon l'invention exige également la mise en œuvre d'au moins un halogénure d'alkyle, c'est-à-dire d'un composé de formule RX dans laquelle X représente un atome de chlore, de brome ou, de préférence un atome d'iode et R est un radical alkyle ayant au maximum 16 atomes de carbone. Bien entendu les halogénures de méthyle qui peuvent être engagés initialement dans le milieu réactionnel, sont susceptibles d'être formés in situ à partir de dérivés halogénés tels que $Cl_2$, $Br_2$, $I_2$, HCl, HBr, HI, $CoBr_2$, $CoI_2$, $RuCl_3$ et $RuI_3$ et du méthanol (matière de départ). En d'autres termes une partie ou la totalité de l'halogénure de méthyle utile à la mise en œuvre du présent procédé peut être formée à partir des précurseurs définis ci-avant.

On constatera en outre que lorsque le dérivé halogéné est choisi parmi les composés du cobalt ou du ruthénium, il peut être considéré comme précurseur non seulement de l'halogénure de méthyle, mais encore comme précurseur du (ou des) catalyseur(s) métallique(s). Dans ce cas particulier il s'avère préférable de charger en outre, initialement, un halogénure d'alkyle et/ou un précurseur de l'halogénure de méthyle, distinct des halogénures métalliques en cause.

L'invention envisage notamment l'utilisation des chlorures, bromures et iodures d'alkyles inférieurs ayant de 1 à 4 atomes de carbone dans la molécule, tels que le bromure et l'iodure de méthyle, le bromure et l'iodure d'éthyle.

De préférence, on utilise l'iodure de méthyle et/ou l'une de ses sources potentielles choisies parmi l'iode, l'acide iodhydrique, l'iodure de cobalt, et l'iodure de ruthénium.

Un avantage supplémentaire de la présente invention réside dans la possibilité d'opérer avec des teneurs en halogène X, provenant de l'halogénure d'alkyle, aussi faibles que 5 millimoles par litre de milieu réactionnel. Il n'est pas souhaitable de dépasser 200 pour cette teneur, notamment pour limiter la corrosion de l'appareillage. De bons résultats sont obtenus avec une teneur de l'ordre de 10 à 100 mMol de X par litre.

Le procédé selon l'invention est réalisé en présence de cobalt. N'importe quelle source de cobalt susceptible de réagir avec l'oxyde de carbone dans le milieu de réaction pour donner des complexes carbonyles du cobalt peut être utilisée dans le cadre de la présente invention.

Des sources typiques de cobalt sont par exemple le cobalt métallique finement divisé, des sels inorganiques tel que le carbonate de cobalt, des sels organiques, en particulier d'un acide gras. Peuvent également être employés les cobalt-carbonyles, hydrocarbonyles, ou leurs complexes. Parmi les dérivés du cobalt concernant pour la mise en œuvre du procédé selon l'invention, on peut citer l'acétate et le formiate de cobalt, les halogénures de cobalt, en particulier l'iodure de cobalt, le dicobaltoctacarbonyle.

Une caractéristique du présent procédé est la mise en œuvre de quantités de cobalt inférieures à 50 milliatomes-grammes par litre de milieu réactionnel. Cette teneur peut être aussi faible que 0,1 mAt-g/l ; elle est de préférence comprise entre 0,5 et 30 mAt-g/l.

Le procédé selon l'invention nécessite également la présence de ruthénium. La forme précise sous laquelle le ruthénium est engagé à la réaction n'est pas fondamentale, dans le cadre de la présente invention. On peut utiliser le ruthénium métallique sous forme finement divisée, ou des composés du ruthénium, tels que $RuCl_3$, $RuI_3$, $RuO_2$, $Ru_3(CO)_{12}$ et $Ru(C_5H_7O_2)_3$.

La quantité de ruthénium à mettre en œuvre dans le cadre du présent procédé doit être au plus de 2 atomes-grammes de ruthénium par atome-gramme de cobalt engagé à la réaction. De préférence, le rapport du ruthénium au cobalt est de préférence compris entre 0,01 et 1.

Le procédé de carbonylation selon la présente invention est conduit de préférence en phase liquide. Comme on opère le plus souvent avec du méthanol en excès, l'emploi simultané d'un solvant supplémentaire est généralement superflu mais on peut en principe faire usage de tels solvants, par exemple d'hydrocarbures, d'esters, d'éther et des produits de la réaction.

Dans le cadre du présent procédé, il n'est pas nécessaire de purifier ou de déshydrater le méthanol au préalable. On peut utiliser du méthanol de qualité technique, renfermant par exemple de l'eau.

Selon le présent procédé, on fait réagir sur le méthanol un mélange de monoxyde de carbone et d'hydrogène. Il est essentiel que ledit mélange renferme au moins 25 % en mole d'hydrogène. En général, on peut utiliser des mélanges renfermant jusqu'à 95 % d'hydrogène. On utilise de préférence des mélanges renfermant de 40 à 80 % d'hydrogène. Le mélange de gaz peut renfermer des impuretés telles que, par exemple, du dioxyde de carbone, de l'oxygène, du méthane et de l'azote.

La réaction est mise en œuvre sous une pression totale généralement comprise entre 50 et 600 bars.

De préférence cette pression est comprise entre 75 et 300 bars, et plus particulièrement entre 100 et 250 bars.

La température de réaction est d'au moins 180 °C environ et peut atteindre 240 °C lorsqu'on opère sans solvant. Lorsqu'on met en œuvre un solvant, ce qui demeure facultatif dans le cadre de la présente invention la température peut atteindre 300 °C. De préférence on opère dans la gamme de température allant de 180 à 230 °C.

Les exemples ci-après illustrent la présente invention sans toutefois en limiter le domaine.

## Exemples

### Mode opératoire

Dans un autoclave en acier inoxydable Z8 - CNDT 17-12 (norme AFNOR) de 250 ml de capacité on charge le méthanol, le cas échéant un solvant et les composants du système catalytique. (Les sources de cobalt et de ruthénium sont, sauf indications contraires, respectivement le dicobaltoctacarbonyle et le triruthénium dodécacarbonyle).

Après fermeture de l'autoclave on établit une pression initiale dont la valeur sera précisée, à l'aide d'un mélange $CO/H_2$ dont la composition sera également indiquée pour chaque essai. L'agitation par un système de va-et-vient est mise en route et l'autoclave est porté à la température voulue en 25 minutes environ, au moyen d'un four annulaire. La pression dans l'autoclave est maintenue par des recharges périodiques d'un mélange $CO/H_2$ dont la composition est sauf indications contraires identiques à celle du mélange ayant servi à établir la pression initiale. Après une certaine durée de réaction à la température indiquée, le chauffage et l'agitation sont arrêtés, l'autoclave est refroidi et dégazé.

Le mélange réactionnel résultant est dilué et analysé par chromatographie gazeuse.

Par les indications g/h × l et g/h × g (Co) on exprimera les productivités en grammes d'acétaldéhyde par heure de réaction respcctivement par litre de volume réactionnel et par gramme de cobalt engagé dans la réaction.

### Exemple 1

Dans l'autoclave et selon le mode opératoire décrit ci-avant, on a chargé : 95 ml de méthanol, 5 ml d'eau, 509 mg (3,58 m Mol) d'iodure de méthyle, 1,8 g (12 m Mole) d'iodure de sodium, 0,126 m At-g de cobalt soit 21,6 mg de dicobaltoctacarbonyle, et 0,117 m At-g de ruthénium soit 25 mg de triruthénium dodécacarbonyle.

On a établi une pression initiale de 140 bars à l'aide d'un mélange $CO/H_2$ équimoléculaire. L'autoclave a été porté à la température de 215 °C. La pression dans l'autoclave a été maintenue entre 230 et 260 bars par des recharges périodiques d'un mélange $CO/H_2$ : 1/2.

En 1 heure 15 minutes de réaction, on a obtenu 24,7 g d'acétaldéhyde soit une productivité de 200 g/h × l et de 2 600 g/h × g (Co).

### Essai témoin a

On a reproduit l'exemple 1 ci-avant en omettant le triruthénium dodécacarbonyle. On a obtenu 14,1 g d'acétaldéhyde, ce qui correspond à une productivité de l'ordre de 110 g/h × 1 et de 1 400 g/h × g (Co). On constate qu'en l'absence de ruthénium la productivité horaire chute considérablement.

### Essai témoin b

En utilisant le mode opératoire décrit précédemment, on a chargé 95 ml de méthanol, 1,5 ml d'eau, 3,58 m Mol d'iodure de méthyle, 12 m Mol d'iodure de sodium et 0,66 m At-g de ruthénium sous forme de triruthénium dodécacarbonyle.

En 1 heure 30 minutes de réaction à 210 °C, la pression dans l'autoclave étant maintenue entre 230 et 260 bars par des recharges périodiques d'un mélange équimoléculaire de CO et $H_2$, on n'a obtenu que 0,7 g d'acétaldéhyde.

On constate que le ruthénium n'a pratiquement aucun effet catalytique dans le procédé envisagé. Lorsqu'on compare l'exemple 1 et l'essai témoin (a) on constate l'effet remarquable produit par de très faibles quantités de ruthénium ajoutées au système catalytique à base de cobalt, effet absolument inattendu lorsque l'on considère les résultats négatifs obtenus dans l'essai témoin (b).

### Exemple 2

Sur une charge constituée par 0,123 m At-g de cobalt, 0,113 m At-g de ruthénium, 12 m Mol de bromure de tétrabutylammonium, 1,5 m Mol d'iodure de méthyle et 100 ml de méthanol on a établi une pression initiale de 140 bars au moyen d'un mélange $CO/H_2$ : 2/3. En 40 minutes de réaction à la

température de 205 °C, la pression dans l'autoclave étant maintenue entre 245 et 260 bars, on a obtenu 5,2 g d'acétaldéhyde. On constate que 78 % du méthanol converti sont transformés en acétaldéhyde ; les sous-produits principaux sont les suivants : éthanol (0,4 g), oxyde de méthyle et d'éthyle (0,4 g) et acétate de méthyle (0,6 g).

Exemple 3

Sur une charge constituée par 100 ml de méthanol, 2,4 m Mol de bromure d'éthyle, 0,108 m At-g de ruthénium, 11 m Mol d'iodure de lithium, et 0,120 m At-g de cobalt on a établi une pression initiale de 140 bars à l'aide d'un mélange $CO/H_2$ : 2/3. En 40 minutes de réaction à la température de 185 °C, la pression dans l'autoclave étant maintenue entre 185 et 245 bars, on a obtenu 10,1 g d'acétaldéhyde. On constate que 84 % (molaire) du méthanol sont transformés en acétaldéhyde.

Exemple 4

Sur une charge constituée par 0,130 m At-g de cobalt, 0,125 m At-g de ruthénium, 12 m Mol d'iodure de triphénylméthylphosphonium, 1,6 m Mol d'iodure de méthyle et 100 ml de méthanol, on a établi une pression initiale de 140 bars au moyen d'un mélange $CO/H_2$ : 2/3. En 40 minutes de réaction à la température de 205 °C, la pression dans l'autoclave étant maintenue entre 230 et 260 bars on a obtenu 19,5 g d'acétaldéhyde, soit une productivité de 290 g/h × l et de 3 800 g/h × g (Co).

On constate que 84 % (molaire) du méthanol converti sont transformés en acétaldéhyde. On observe la présence d'une quantité d'éthanol inférieure à 1 g.

Exemple 5

Sur une charge constituée par 100 ml de méthanol, 1,17 m Mol d'iodure d'éthyle, 12 m Mol d'iodure de sodium, 0,116 m At-g de ruthénium et 0,128 m At-g de cobalt, on a établi une pression initiale de 140 bars à l'aide d'un mélange $CO/H_2$ équimoléculaire. En 40 minutes de réaction à température de 205 °C, la pression dans l'autoclave étant maintenue entre 230 et 260 bars, on a obtenu 20,7 g d'acétaldéhyde soit une productivité de 310 g/h × l et de 4 100 g/l × g (Co).

On constate que 81 % (molaire) du méthanol converti, sont transformés en acétaldéhyde.

Exemple 6

Sur une charge constituée par 0,126 m At-g de cobalt, 0,121 m At-g de ruthénium, 12 m Mol d'iodure de sodium, 1,72 m Mol d'iodure de méthyle et 100 ml de méthanol, on a établi une pression initiale de 140 bars au moyen d'un mélange $CO/H_2$ équimoléculaire. En 1 heure 15 minutes de réaction à la température de 215 °C, la pression dans l'autoclave étant maintenue entre 230 et 260 bars on a obtenu 27,7 g d'acétaldéhyde.

On constate que 73 % (molaire) du méthanol converti sont transformés en acétaldéhyde.

Exemples 7 à 11

Sur une charge renfermant 100 ml de méthanol, 12 m Mol d'iodure de tributylméthyl phosphonium, de l'iodure de méthyle, du dicobalt octacarbonyle et du triruthénium dodécacarbonyle, on a établi une pression initiale de 70 bars pour les exemples 10 et 11, et de 40 bars pour les autres exemples ainsi que pour l'essai témoin c, au moyen d'un mélange $CO/H_2$ : 2/3. La durée de réaction à 250 °C était de 40 minutes, la pression dans l'autoclave étant maintenue entre 140 et 155 bars pour les exemples 10 et 11, et entre 95 et 105 bars pour les autres exemples ainsi que pour l'essai témoin (c). Les conditions particulières ainsi que les résultats obtenus sont portés dans le tableau unique ci-après.

On observe dans tous les cas que de 80 à 90 % (molaire) du méthanol converti sont transformés en acétaldéhyde.

On constate que le système catalytique particulier conduit à des résultats remarquables, malgré les faibles pressions mises en œuvre.

(Voir Tableau p. 8).

Tableau unique

| n° d'exemple | cobalt m At - g | Ruthénium m At - g | $CH_3$ I m Mol | Acétaldéhyde (g) | Productivité | |
|---|---|---|---|---|---|---|
| | | | | | en g/h x 1 | en g/h x g(Co) |
| C | 0,56 | 0 | 1,57 | 2,0 | 30 | 90 |
| 7 | 0,55 | 0,057 | 1,44 | 6,0 | 90 | 280 |
| 8 | 0,56 | 0,237 | 1,59 | 6,3 | 96 | 300 |
| 9 | 1,09 | 0,114 | 1,75 | 7,5 | 110 | 180 |
| 10 | 1,13 | 0,115 | 1,75 | 10,9 | 160 | 250 |
| 11(*) | 1,10 | 0,112 | 3 | 11,5 | 175 | 270 |

(*) exemple réalisé avec l'iodure de sodium (en remplacement de l'iodure de méthyltributylphosphonium).

## Revendications

1. Procédé de préparation de l'acétaldéhyde par hydrocarbonylation du méthanol, en phase liquide, à une température d'au moins 180 °C, sous une pression totale comprise entre 50 et 600 bars, en présence de cobalt et de ruthénium caractérisé en ce que la réaction est conduite en présence d'au moins un halogénure ionique dont le cation est choisi dans le groupe constitué par les cations des métaux alcalins, les cations des métaux alcalino-terreux, les cations ammonium quaternaire et les cations phosphonium quaternaire, et d'au moins un halogénure d'alkyle, le rapport molaire $X^-/Co$, $X^-$ provenant de l'halogénure ionique, étant supérieur ou égal à 5, la teneur en halogène X, X provenant de l'halogénure d'alkyle, étant supérieure ou égale à 5 m Mol par litre de milieu réactionnel, la concentration en cobalt étant d'au plus de 50 m At-g par litre de milieu réactionnel, et le rapport molaire Ru/Co étant au plus égal à 2.

2. Procédé selon la revendication 1, caractérisé en ce que l'halogénure d'alkyle est choisi parmi les chlorures, les bromures et les iodures d'alkyles ayant de 1 à 4 atomes de carbone dans la molécule.

3. Procédé selon la revendication 2, caractérisé en ce que l'halogénure d'alkyle est un halogénure de méthyle.

4. Procédé selon la revendication 3, caractérisé en ce que l'halogénure de méthyle est, pour tout ou partie produit in situ à partir d'au moins un composé choisi dans le groupe constitué par le chlore, le brome et l'iode moléculaires, les acides halohydriques correspondants, le bromure et l'iodure de cobalt, le bromure et l'iodure de ruthénium.

5. Procédé selon l'une quelconque des revendications précédentes, caractérisé en ce que le cation de l'halogénure ionique est choisi parmi les cations alcalins et les cations alcalino-terreux.

6. Procédé selon l'une quelconque des revendications précédentes, caractérisé en ce que l'halogénure ionique est un iodure.

7. Procédé selon l'une quelconque des revendications précédentes, caractérisé en ce que l'halogénure d'alkyle est un iodure.

8. Procédé selon l'une quelconque des revendications précédentes, caractérisé en ce que le rapport $X^-/Co$ est compris entre 5 et 150.

9. Procédé selon l'une quelconque des revendications précédentes, caractérisé en ce que la teneur en halogène X, X provenant de l'halogénure d'alkyle, est comprise entre 10 et 100 m Mol par litre de milieu réactionnel.

10. Procédé selon l'une quelconque des revendications précédentes, caractérisé en ce que la concentration du cobalt est comprise entre 0,5 et 30 milliatomes-grammes par litre de milieu réactionnel.

11. Procédé selon l'une quelconque des revendications précédentes, caractérisé en ce que le rapport molaire Ru/Co est compris entre 0,01 et 1.

12. Procédé selon l'une quelconque des revendications précédentes, caractérisé en ce que la pression totale est comprise entre 75 et 300 bars, et de préférence, entre 100 et 200 bars.

13. Procédé selon l'une quelconque des revendications précédentes, caractérisé en ce que la température est comprise entre 180 et 230 °C.

## Claims

1. Process for the preparation of acetaldehyde by the hydrocarbonylation of methanol in the liquid phase, at a temperature of at least 180 °C, under a total pressure of between 50 and 600 bars, in the presence of cobalt and ruthenium, characterised in that the reaction is carried out in the presence of at

least one ionic halide of which the cation is chosen from the group comprising alkali metal cations, alkaline earth metal cations, quaternary ammonium cations and quaternary phosphonium cations, and of at least one alkyl halide, the molar ratio $X^-/Co$, $X^-$ originating from the ionic halide, being greater than or equal to 5, the proportion of halogen X, X originating from the alkyl halide, being greater than or equal to 5 millimols per litre of reaction medium, the cobalt concentration being at most 50 mg atoms per litre of reaction medium and the molar ratio Ru/Co being equal to at most 2.

2. Process according to Claim 1, characterised in that the alkyl halide is chosen from amongst alkyl chlorides, bromides and iodides having from 1 to 4 carbon atoms in the molecule.

3. Process according to Claim 2, characterised in that the alkyl halide is a methyl halide.

4. Process according to Claim 3, characterised in that all or part of the methyl halide is produced in situ from at least one compound chosen from the group comprising molecular chlorine, bromine and iodine, the corresponding hydrohalic acids, cobalt bromide and iodide and ruthenium bromide and iodide.

5. Process according to any one of the preceding claims, characterised in that the cation of the ionic halide is chosen from amongst alkali metal cations and alkaline earth metal cations.

6. Process according to any one of the preceding claims, characterised in that the ionic halide is an iodide.

7. Process according to any one of the preceding claims, characterised in that the alkyl halide is an iodide.

8. Process according to any one of the preceding claims, characterised in that the ratio $X^-/Co$ is between 5 and 150.

9. Process according to any one of the preceding claims, characterised in that the proportion of halogen X, X originating from the alkyl halide, is between 10 and 100 millimols per litre of reaction medium.

10. Process according to any one of the preceding claims, characterised in that the cobalt concentration is between 0.5 and 30 milligram atoms per litre of reaction medium.

11. Process according to any one of the preceding claims, characterised in that the molar ratio Ru/Co is between 0.01 and 1.

12. Process according to any one of the preceding claims, characterised in that the total pressure is between 75 and 300 bars and preferably between 100 and 200 bars.

13. Process according to any one of the preceding claims, characterised in that the temperature is between 180 and 230 °C.

**Ansprüche**

1. Verfahren zur Herstellung von Acetaldehyd mittels Hydrocarbonylieren von Methanol in flüssiger Phase bei einer Temperatur von mindestens 180 °C unter einem Gesamtdruck von 50 bis 600 bar, in Gegenwart von Kobalt und Ruthenium, dadurch gekennzeichnet, daß die Reaktion ausgeführt wird in Gegenwart mindestens eines ionischen Halogenids, dessen Kation ausgewählt wird aus der Gruppe bestehend aus den Kationen der Alkalimetalle, der Kationen der Erdalkalimetalle, der quaternären Ammonium- und Phosphoniumkationen, sowie mindestens eines Alkylhalogenids, wobei das Molverhältnis $X^-/Co$, $X^-$ stammt aus dem ionischen Halogenid, gleich oder größer 5 ist, der Gehalt an Halogen X, X stammt aus dem Alkylhalogenid, 5 mmol/l Reaktionsmedium oder mehr beträgt, die Konzentration an Kobalt höchstens 50 mgAtom/l Reaktionsmedium und das Molverhältnis Ru/Co höchstens 2 beträgt.

2. Verfahren nach Anspruch 1, dadurch gekennzeichnet, daß das Alkylhalogenid ausgewählt wird aus den Alkylchloriden, Alkylbromiden und Alkyljodiden, die 1 bis 4 Kohlenstoffatome im Molekül enthalten.

3. Verfahren nach Anspruch 2, dadurch gekennzeichnet, daß das Alkylhalogenid ein Methylhalogenid ist.

4. Verfahren nach Anspruch 3, dadurch gekennzeichnet, daß das Methylhalogenid ganz oder teilweise in situ hergestellt wird, ausgehend von mindestens einer Verbindung ausgewählt aus der Gruppe bestehend aus molekularem Chlor, molekularem Brom und molekularem Jod, den entsprechenden Halogenwasserstoffsäuren, Kobaltbromid und Kobaltjodid sowie Rutheniumbromid und Rutheniumjodid.

5. Verfahren nach einem der vorangegangenen Ansprüche, dadurch gekennzeichnet, daß das Kation des ionischen Halogenids ausgewählt wird aus den Alkalikationen und den Erdalkalikationen.

6. Verfahren nach einem der vorangegangenen Ansprüche, dadurch gekennzeichnet, daß das ionische Halogenid ein Jodid ist.

7. Verfahren nach einem der vorangegangenen Ansprüche, dadurch gekennzeichnet, daß das Alkylhalogenid ein Jodid ist.

8. Verfahren nach einem der vorangegangenen Ansprüche, dadurch gekennzeichnet, daß das Verhältnis $X^-/Co$ 5 bis 150 beträgt.

9. Verfahren nach einem der vorangegangenen Ansprüche, dadurch gekennzeichnet, daß der Gehalt an Halogen X, wobei X aus dem Alkylhalogenid stammt, 10 bis 100 mmol/l Reaktionsmedium beträgt.

10. Verfahren nach einem der vorangegangenen Ansprüche, dadurch gekennzeichnet, daß die Konzentration an Kobalt 0,5 bis 30 mgAtom/l Reaktionsmedium beträgt.

11. Verfahren nach einem der vorangegangenen Ansprüche, dadurch gekennzeichnet, daß das Molverhältnis Ru/Co 0,01 bis 1 beträgt.

12. Verfahren nach einem der vorangegangenen Ansprüche, dadurch gekennzeichnet, daß der Gesamtdruck 75 bis 300 bar, vorzugsweise 100 bis 200 bar beträgt.

13. Verfahren nach einem der vorangegangenen Ansprüche, dadurch gekennzeichnet, daß die Temperatur 180 bis 230 °C beträgt.